(19) Europäisches Patentamt
European Patent Office
Office européen des brevets

(11) Veröffentlichungsnummer: **0 467 084 A1**

(12) # EUROPÄISCHE PATENTANMELDUNG

(21) Anmeldenummer: **91110036.0**

(51) Int. Cl.5: **A61B 17/22**, A61N 5/01

(22) Anmeldetag: **19.06.91**

(30) Priorität: **14.07.90 DE 4022496**

(43) Veröffentlichungstag der Anmeldung:
**22.01.92 Patentblatt 92/04**

(84) Benannte Vertragsstaaten:
**CH DE FR GB IT LI NL SE**

(71) Anmelder: **DORNIER MEDIZINTECHNIK GMBH
Postfach 1128
W-8034 Germering 1(DE)**

(72) Erfinder: **Artmeier, Theo
Tannenfleckstrasse 12 B
W-8038 Gröbenzell(DE)**

(74) Vertreter: **Kasseckert, Rainer
DORNIER GMBH Kleeweg 3
W-7990 Friedrichshafen 1(DE)**

(54) **Kinematik eines Lithotripters.**

(57) Vorrichtung zur Behandlung von Patienten mit fokussierten Stosswellen, insbesondere Lithotripter, mit einem Therapiekopf der mittels eines Armes an einem Gehäuse befestigt ist und Mittel zum Erzeugen, Fokussieren und Einleiten von Stosswellen in einen Patientenkörper enthält, wobei der Therapiekopf (**TK**) am Arm (**8**) mittels eines Getriebes (**10**) befestigt ist.

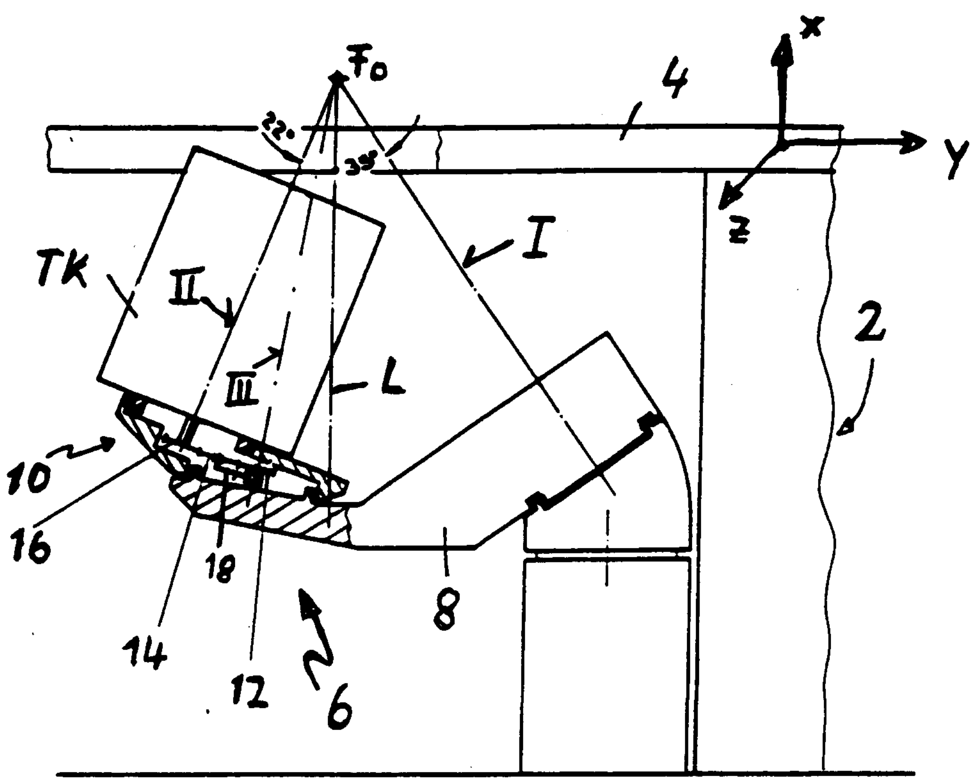

Fig. 1



Rank Xerox (UK) Business Services

Die Erfindung betrifft eine Vorrichtung zur Behandlung von Patienten mit fokussierten Stosswellen, insbesondere Lithotripter, mit einem Therapiekopf der mittels eines Armes an einem Gehäuse befestigt ist und Mittel zum Erzeugen, Fokussieren und Einleiten von Stosswellen in einen Patientenkörper enthält, wobei die Achse des Therapiekopfes auf einem Kegelmantel beweglich ist, dessen Spitze im Fokus des Therapiekopfes liegt (Isozentrum). Ein Gerät dieser Art ist aus der **EP 0 286 170** bekannt.

Aufgabe der Erfindung ist es, eine solche Vorrichtung bezüglich der Ortung, der Positionierung und Behandlung des Patienten kinematisch zu verbessern.

Diese Aufgabe wird erfindungsgemäss gelöst von einer Vorrichtung mit den Merkmalen des Anspruchs 1. Ausführungsformen der Erfindung sind in den Unteransprüchen beschrieben.

Der Therapiekopf ist so angeordnet, dass er auf einem Kegelmantel beweglich ist, wobei die Spitze des Kegelmantels mit dem Fokus des Therapiekopfes zusammenfällt (isozentrische Bewegung). Der Kegelmantel liegt erfindungsgemäss so, dass eine seiner Erzeugenden senkrecht verläuft, das heisst, dass der Therapiekopf auch in eine Stellung gebracht werden kann, in der seine Längsachse senkrecht verläuft, so dass auch die Behandlung von Gallensteinen möglich ist.
Ein Getriebe verschwenkt den Therapiekopf auf dem Kegelmantel so, dass er bezüglich des Gesamtgeräts keine Drehung um seine Längsachse durchführt. Damit werden unbeabsichtigte Verschiebungen bei Ortungsaufnahmen verhindert.

In einer Ausführung ist am Therapiekopf ein Ultraschall-Ortungsgerät so befestigt, dass dessen Mittelachse ständig auf den Brennpunkt des Therapiekopfes zielt. Dies hat den Vorteil, dass auf dem Ultraschallbild in der Mittellinie immer der Fokus liegt, wodurch die Ortung wesentlich vereinfacht wird. Der Arzt hat eine relativ grosse Freiheit, Fenster für das Ultraschallbild am Patientenkörper zu suchen und verliert dabei nie den Fokus aus der Bildmitte.

Die Erfindung wird anhand von Figuren näher erläutert.

Es zeigen:

| | |
|---|---|
| Fig. 1 | eine Prinzipskizze der erfindungsgemässen Vorrichtung mit einem Getriebe in Schnittdarstellung, |
| Fig. 2 | das Getriebe von Fig. 1 in Draufsicht, |
| Fig. 3 und 4 | Behandlungsstellung des Therapiekopfs "Konkrement in rechter Niere", |
| Fig. 5 und 6 | Behandlungsstellung des Therapiekopfs "Konkrement in linker Niere", |
| Fig. 7 | Behandlungsstellung des Therapiekopfs "Konkrement in Galle" und |
| Fig. 8 | eine vergrößerte Darstellung von Einzelheiten der Fig. 1. |

Das in Fig. 1 gezeigte Gerät zur Behandlung von Konkrementen, besteht aus einem Gehäuse 2, einer Patientenliege **4**, die mit Ausnehmungen versehen ist und in x-, y-, z-Richtung motorisch oder manuell verschiebbar ist, sowie aus einer Behandlungsvorrichtung **6**, die nachfolgend näher beschrieben wird.

Am Gehäuse **2** des Behandlungsgeräts ist ein Arm **8** drehbar befestigt, der auch in Fig. 8 dargestellt ist. Die Drehachse **I** verläuft unter einem Winkel von 35° zur Lotrechten (**L**). Der Arm **8** ist abgewinkelt und trägt an seinem anderen Ende einen Therapiekopf **TK** , wobei sich zwischen dem Arm **8** und dem Therapiekopf **TK** ein Getriebe **10** befindet. Das Getriebe besteht aus drei Zahnrädern **12, 14, 16,** wobei Zahnrad **12** starr am Arm **8** befestigt ist und Zahnrad **16** starr mit dem Therapiekopf **TK** verbunden ist. Ausgleichszahnrad **14** ist mittels einer Kurbel **18** am Arm **8** gehalten.

In Fig. 2 ist ein Teil der Bahn **20** gezeigt, auf der sich der Therapiekopf **TK** bewegt, wenn er verschwenkt wird. Bei der Bewegung auf der Bahn **20** bewirkt das Getriebe **10,** dass der Therapiekopf **TK** keine Rotation um seine Achse **II** ausführt, wenn die Zahnräder **12, 14, 16** hinsichtlich ihrer Zähnezahlen und Teilkreisdurchmesser untereinander gleich sind. Das Getriebe bewirkt somit, dass eine in den Therapiekopf integrierte fächerförmige Ultraschall-Ortungseinrichtung (nicht dargestellt) ständig ohne Richtungsänderung durch den Fokus Fo (Isozentrum) verläuft. Das Getriebe **10** verhindert eine Rotation des Therapiekopfes **TK** um seine Achse **II**.

Wird der Therapiekopf **TK** um die Achse **III** bewegt, kann der Therapiekopf in eine Lage senkrecht unter einen Patienten gebracht werden, wie dies in Fig. 7 gezeigt ist. Wie aus Fig. 1 und 8 hervorgeht, beträgt der Kegelwinkel von Drehachse **II** 22°. Bei diesem Winkel werden zusammen mit dem Winkel von 35° zur Lotrechten (**L**) nahezu rechtwinklige Ankoppelungen an die rechte und linke Niere ermöglicht, wie dies in Fig. 3 und 5 dargestellt ist.

Der dort gezeigte Winkel von 55° ergibt sich als Summe aus den beiden vorgenannten Winkeln. Zur Ankopplung ist in Fig. 3 und 5 auch ein Faltenbalg **22** gezeigt, der elastisch gegen den Körper **24** eines Patienten gefahren wird. Die Therapiestellung von Fig. 3 und 5 sind auch in Fig. 4 und 6 in Draufsicht gezeigt.

Mittels des Getriebes **10** kann der Therapiekopf **TK** auch in eine Lage senkrecht unter einen Patienten gebracht werden, wie dies in Fig. 7 gezeigt ist.

## Patentansprüche

1. Vorrichtung zur Behandlung von Patienten mit fokussierten Stosswellen, insbesondere Lithotripter, mit einem Therapiekopf der mittels eines Armes an einem Gehäuse befestigt ist und Mittel zum Erzeugen, Fokussieren und Einleiten von Stosswellen in einen Patientenkörper enthält, wobei die Achse des Therapiekopfes auf einem Kegelmantel beweglich ist, dessen Spitze im Fokus des Therapiekopfes liegt (Isozentrum), **dadurch gekennzeichnet,** dass der Therapiekopf (**TK**) am Arm (**8**) mittels eines Getriebes (**10**) befestigt ist.

2. Vorrichtung nach Anspruch 1, dadurch gekennzeichnet, dass die Drehachse (**I**) des Armes (**8**) am Gehäuse (**2**) durch das Isozentrum (**Fo**) verläuft.

3. Vorrichtung nach Ansprüchen 1 oder 2, **dadurch gekennzeichnet,** dass das Getriebe (**10**) ein Zahnradgetriebe ist, das aus einem mit dem Arm (**8**) fest verbundenem Zahnrad (**12**), einem mittels einer Kurbel (**18**) am Arm (**8**) befestigtem drehbaren Ausgleichszahnrad (**14**) und einem am Therapiekopf (**TK**) befestigten Zahnrad (**16**) besteht.

4. Vorrichtung nach Anspruch 3, **dadurch gekennzeichnet,** dass die Zähnezahl und der Teilkreisdurchmesser der Zahnräder (**12, 14, 16**) des Getriebes (**10**) untereinander gleich sind.

5. Vorrichtung nach Anspruch 4, **dadurch gekennzeichnet,** dass das Getriebegehäuse (**10**) mittels Lagern (Wälz- oder Gleitlagern) am Arm (**8**) und am Therapiekopf (**TK**) gelagert ist.

6. Vorrichtung nach Ansprüchen 1 oder 3, **dadurch gekennzeichnet,** dass die Drehachse (**II**) des Therapiekopfes (**TK**) und die Achse des mit dem Arm (**8**) verbundenem Zahnrads (**12**) einen Winkel von ca. 11° miteinander bilden und die Drehachse (**I**) des Armes (**8**) am Gehäuse (**2**) zur Lotrechten (**L**) einen Winkel von 35° aufweist.

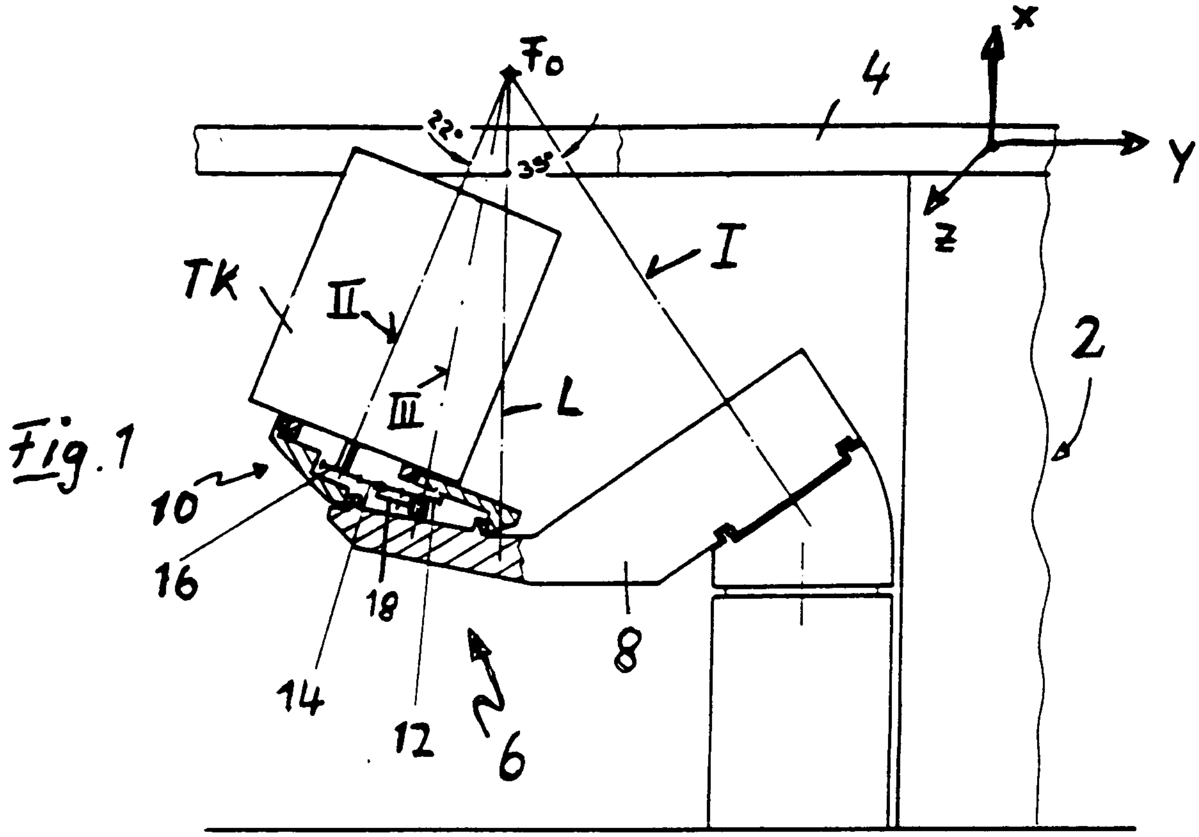
Fig. 1
Fo
22°
35°
4
X
Y
Z
I
TK
II
III
L
2
10
16
18
14
12
6
8

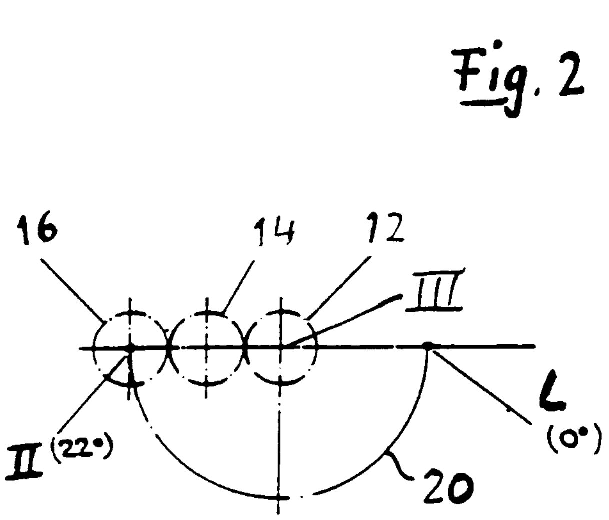
Fig. 2
16
14
12
III
II (22°)
L (0°)
20

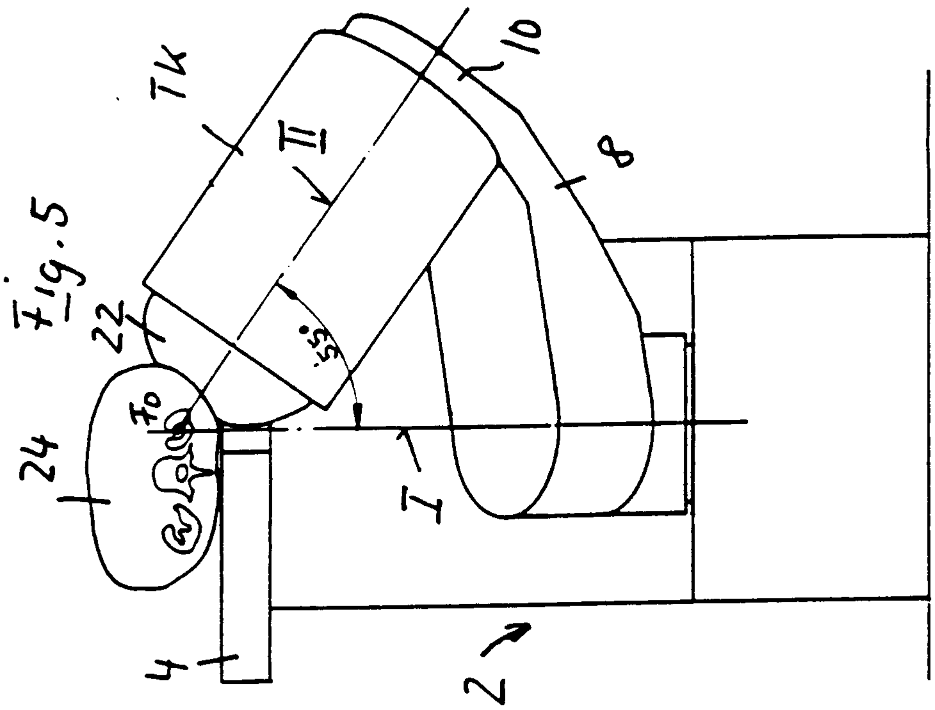

Fig. 5

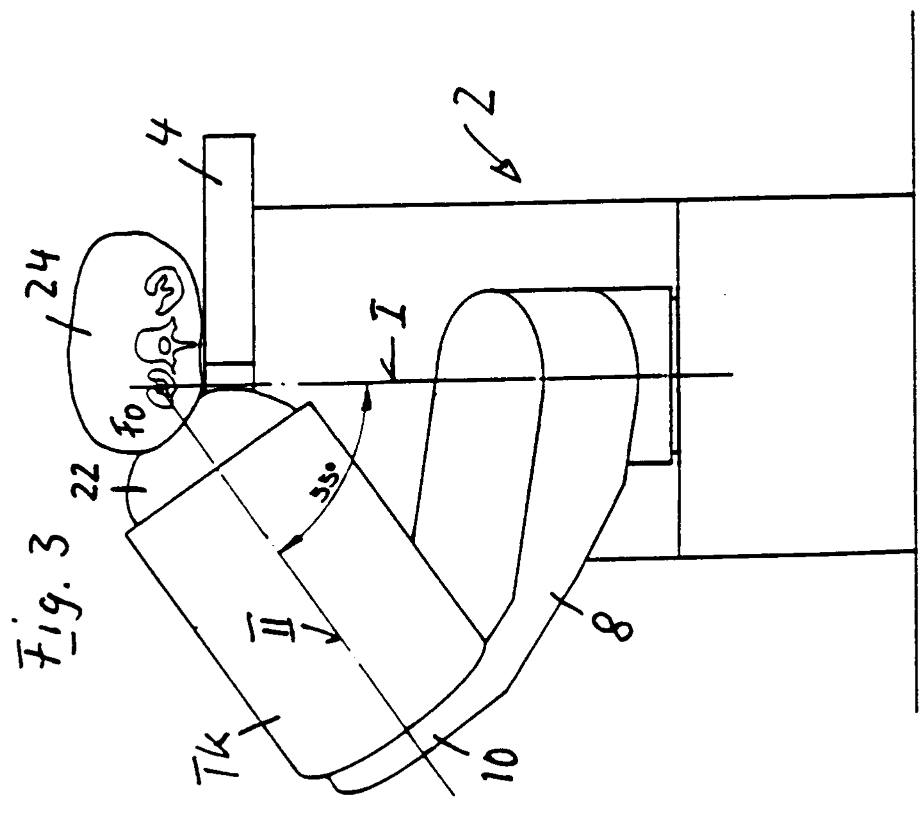

Fig. 3

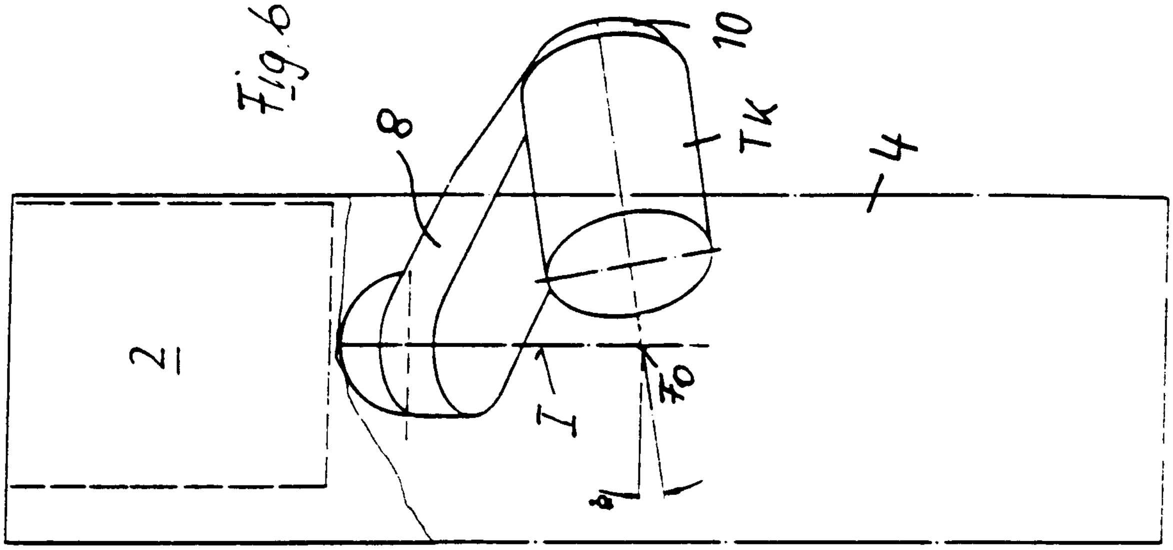


Fig. 6

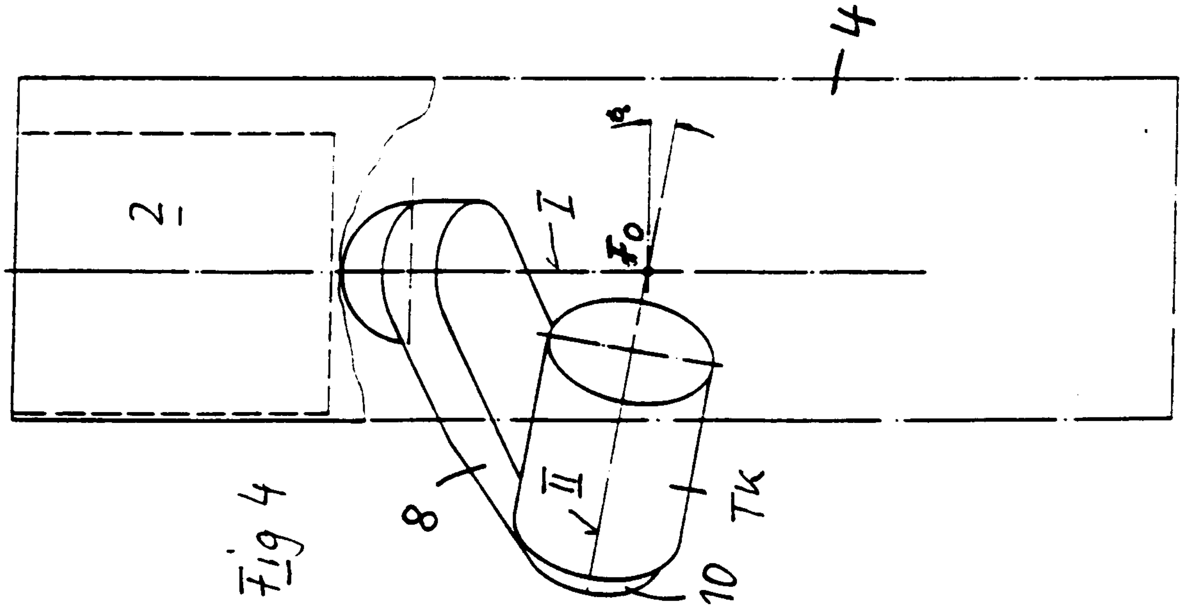


Fig 4

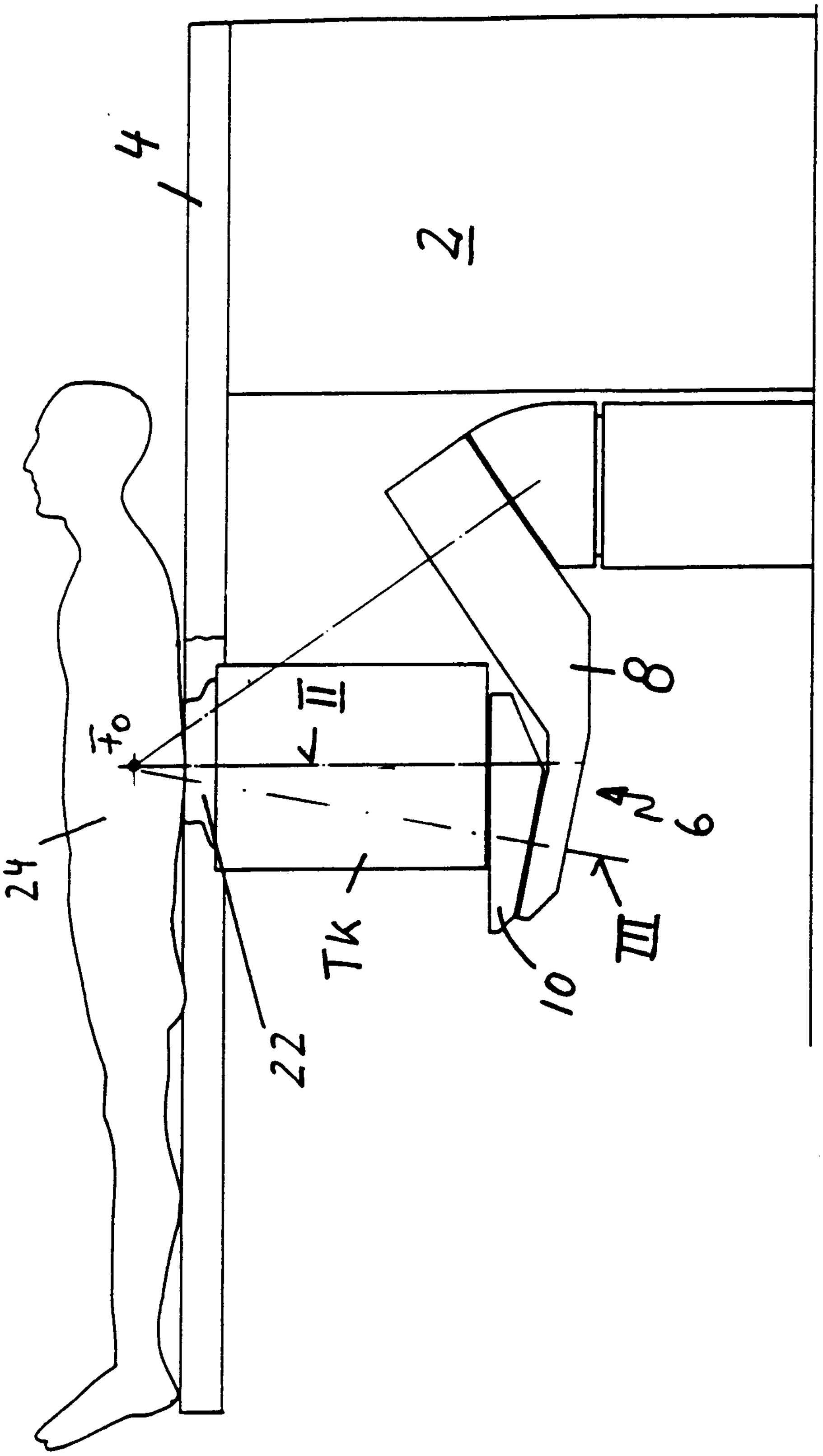


Fig. 7

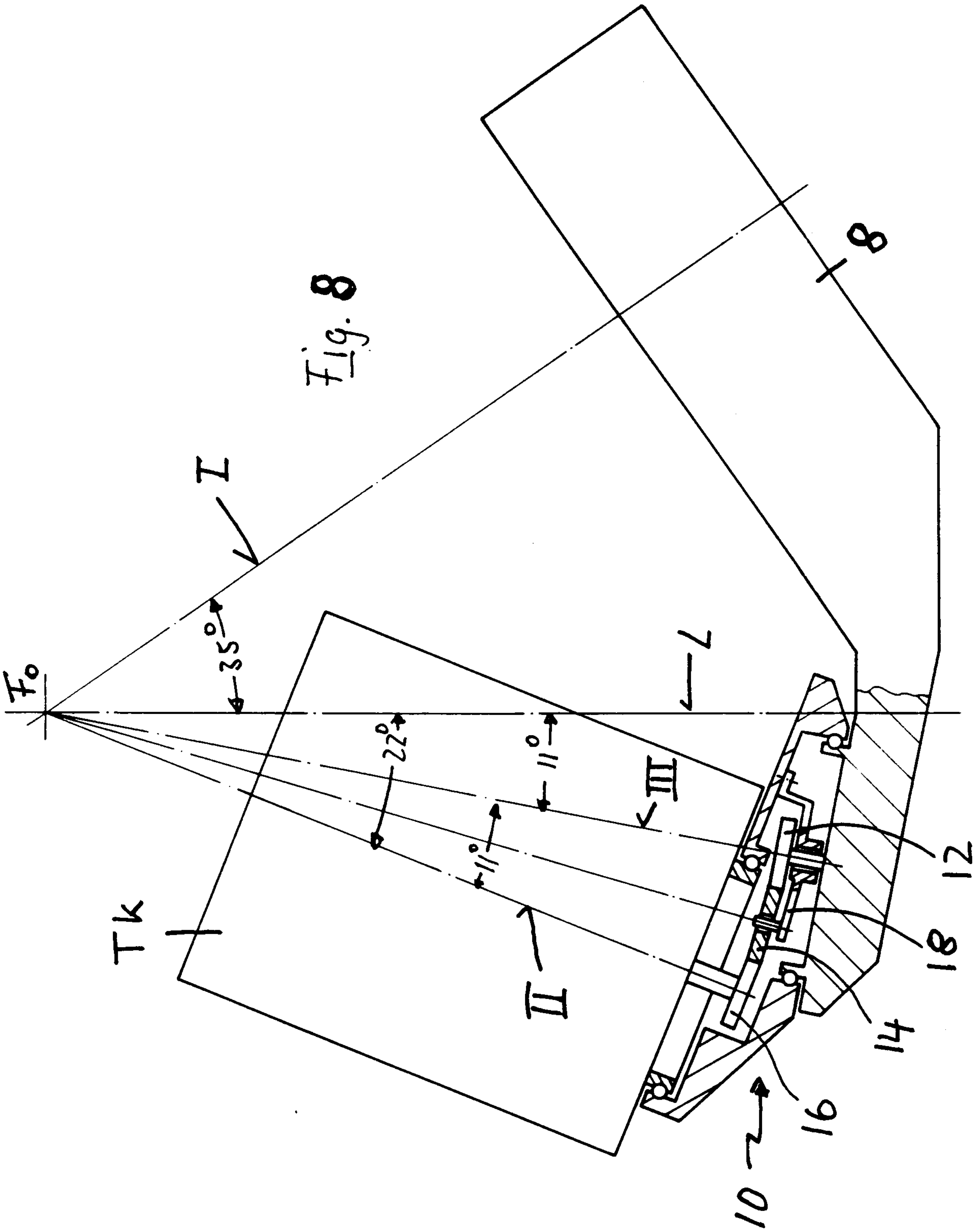


Fig. 8

Europäisches Patentamt

# EUROPÄISCHER RECHERCHENBERICHT

Nummer der Anmeldung

**EP 91 11 0036**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 286 170 (PHILIPS)<br>* Spalte 3, Zeile 18 - Zeile 53; Abbildung 1 *<br>– – – | 1-6 | A 61 B 17/22<br>A 61 N 5/01 |
| Y | BE-A-551 149 (PHILIPS)<br>* Seite 10, Zeile 12 - Seite 11, Zeile 14; Abbildung 5 *<br>– – – – – | 1-6 | |

RECHERCHIERTE SACHGEBIETE (Int. Cl.5)

A 61 B
A 61 N

Der vorliegende Recherchenbericht wurde für alle Patentansprüche erstellt

| Recherchenort | Abschlußdatum der Recherche | Prüfer |
|---|---|---|
| Den Haag | 17 Oktober 91 | MOERS R.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument